# EUROPEAN PATENT APPLICATION

(11) **EP 1 953 555 A1**
(43) Date of publication of application: **06.08.2008**
(21) Application number: 08001805.4
(22) Date of filing: 31.01.2008
(51) Int. Cl.: G01N 33/543, G01N 33/548, G01N 33/553

(54) **Physiologically active substance-immobilized substrate**

(30) Priority: 31.01.2007 JP 2007020350
(71) Applicant: Fujifilm Corporation, Tokyo 106-0031 (JP)
(72) Inventor: Saitoh, Yukou, Ashigarakami-gu Kanagawa 258-8577 (JP); Ezoe, Toshihide, Woodbridge, CT 06525 (US)
(74) Representative: HOFFMANN EITLE

(57) **Abstract**

It is an object of the present invention to provide a physiologically active substance-immobilized substrate wherein the stability of the physiologically active substance has been improved by adding a compound having the effect of suppressing deactivation to the substrate on which the physiologically active substance has been immobilized. The present invention provides a substrate which has, on the surface thereof, a physiologically active substance that has been immobilized thereon via a hydrophilic polymer layer formed with hydrophilic polymers, and Compound having a mean molecular weight between 350 and 5,000,000 and having a residue capable of forming a hydrogen bond.

## Description

### Technical Field

The present invention relates to a physiologically active substance-immobilized substrate having excellent preservation stability. More specifically, the present invention relates to a physiologically active substance-immobilized substrate, wherein the stability of the physiologically active substance has been improved by adding a compound having the effect of suppressing deactivation to the substrate on which the physiologically active substance has been immobilized.

### Background Art

Recently, a large number of measurements using intermolecular interactions such as immune responses are being carried out in clinical tests, etc. However, since conventional methods require complicated operations or labeling substances, several techniques are used that are capable of detecting the change in the binding amount of a test substance with high sensitivity without using such labeling substances. Examples of such a technique may include a surface plasmon resonance (SPR) measurement technique, a quartz crystal microbalance (QCM) measurement technique, and a measurement technique of using functional surfaces ranging from gold colloid particles to ultra-fine particles. The SPR measurement technique is a method of measuring changes in the refractive index near an organic functional film attached to the metal film of a chip by measuring a peak shift in the wavelength of reflected light, or changes in amounts of reflected light in a certain wavelength, so as to detect adsorption and desorption occurring near the surface. The QCM measurement technique is a technique of detecting adsorbed or desorbed mass at the ng level, using a change in frequency of a crystal due to adsorption or desorption of a substance on gold electrodes of a quartz crystal (device). In addition, the ultra-fine particle surface (nm level) of gold is functionalized, and physiologically active substances are immobilized thereon. Thus, a reaction to recognize specificity among physiologically active substances is carried out, thereby detecting a substance associated with a living organism from sedimentation of gold fine particles or sequences.

In all of the above-described techniques, interaction between biomolecules is analyzed by measuring a specific binding reaction between a physiologically active substance and a test substance. Therefore, the surface where a physiologically active substance is immobilized is important.

A physiologically active substance is immobilized on the surface of a substrate via a covalent bond, a ligand bond, an ionic bond, physical adsorption, an inclusion method, etc. However, such immobilization method has been problematic in that the physiologically active substance immobilized on the surface of the substrate deteriorates as the time has passed, and in that a specific binding reaction decreases. It is considered that such deterioration occurs over time due to various factors that complicatedly intertwine with one another. Such deterioration particularly significantly occurs when the physiologically active substance is in a dry state. Thus, in order to prevent the physiologically active substances from being dried, a hydrophilic polymer (e.g. JP Patent Publication (Kokai) No. 2006-170832 A) or sugars such as a monosaccharide or a disaccharide (e.g. JP Patent Publication (Kokai) No. 2005-300401 and U.S. Patent Publication No. 2003-0175827) have been added to a solution that has contained the physiologically active substance, thereby making an attempt to prevent deterioration. However, a sufficient effect could not be obtained by such attempt.

### Disclosure of the Invention

It is an object of the present invention to solve the aforementioned problem of the prior art techniques. That is to say, the present invention relates to a physiologically active substance-immobilized substrate having excellent preservation stability. More specifically, it is an object of the present invention to provide a physiologically active substance-immobilized substrate wherein the stability of the physiologically active substance has been improved by adding a compound having the effect of suppressing deactivation to the substrate on which the physiologically active substance has been immobilized.

As a result of intensive studies, the present inventors have found that the aforementioned object can be achieved by adding a compound having a mean molecular weight between 350 and 5,000,000 and also having a residue capable of forming a hydrogen bond (hereinafter referred to as "Compound S") to a substrate on which a physiologically active substance has been immobilized via a hydrophilic polymer layer formed with hydrophilic polymers, thereby completing the present invention.

That is to say, the present invention provides a substrate which has, on the surface thereof, a physiologically active substance that has been immobilized thereon via a hydrophilic polymer layer formed with hydrophilic polymers, and Compound having a mean molecular weight between 350 and 5,000,000 and having a residue capable of forming a hydrogen bond.

Preferably, the mean molecular weight of Compound S is between 1,200 and 70,000.

Preferably, Compound S is a polysaccharide.

Preferably, the polysaccharide is composed of 20 to 600 monosaccharides.

Preferably, Compound S has a dextran skeleton and has a mean molecular weight between 10,000 and 2,000,000.

Preferably, Compound S is a nonionic compound.

Preferably, Compound S has a polyethylene oxide skeleton.

Preferably, the physiologically active substance is a protein.

Preferably, the protein is protein A, protein G, avidins, calmodulin, or an antibody.

Preferably, the substrate has a layer that contains Compound S on a layer that contains the physiologically active substance.

Preferably, the skeleton of the hydrophilic polymers that form the hydrophilic polymer layer is substantially identical to the skeleton of Compound S.

Preferably, the ratio of the mean molecular weight of Compound S to the mean molecular weight of the hydrophilic polymers that form the hydrophilic polymer layer is between 0.005 and 0.2.

Preferably, the physiologically active substance is immobilized on the hydrophilic polymer layer via a covalent bond.

Preferably, the physiologically active substance is immobilized on the aforementioned layer by activating the hydrophilic polymers that form the hydrophilic polymer layer.

Preferably, the substrate has a metal film, and the hydrophilic polymer layer binds to the metal film via a self-assembled membrane-forming molecule represented by the following formula A-1:

**HS(CH₂)ₙX** **A-1**

Preferably, the refractive index of a material for the substrate is between 1.4 and 1.7.

Preferably, the substrate is used in non-electrochemical detection.

Preferably, the substrate is used in surface plasmon resonance analysis.

In another aspect, the present invention provides a sensor unit comprising the substrate of the present invention, or a sensor device comprising the sensor unit.

In a further aspect, the present invention provides a method for producing a substrate, on the surface of which a physiologically active substance with improved stability has been immobilized, which comprises: a step of allowing a solution containing a physiologically active substance to come into contact with a hydrophilic polymer layer on the surface of a substrate and then drying it, so as to immobilize the physiologically active substance on the surface of the substrate; and a step of allowing an aqueous solution containing Compound S to come into contact with the substrate surface after immobilization of the physiologically active substance.

Preferably, the concentration of the solution containing the above-described physiologically active substance is between 0.1 mg/ml and 10 mg/ml.

Preferably, the concentration of Compound S in the solution containing the above-described compound is between 0.1% by weight and 5% by weight.

In a further aspect, the present invention provides an agent for suppressing deactivation of a physiologically active substance, which comprises a compound having a dextran skeleton or a polyethylene oxide skeleton and having a mean molecular weight between 1,200 and 70,000.

According to the present invention, it became possible to provide a substrate wherein stability of a physiologically active substance immobilized on the surface of the substrate has been improved.

### Brief Description of the Invention

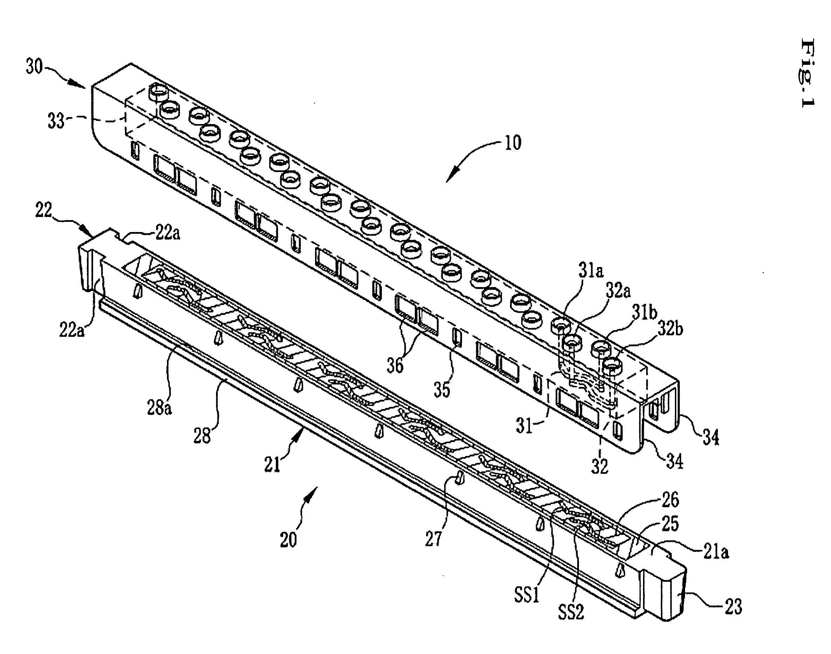

Figure 1 shows an exploded perspective view of a sensor unit

### Preferred Embodiments of the Invention

Hereinafter, the embodiments of the present invention will be explained.

The substrate of the present invention, on the surface of which a physiologically active substance with improved stability has been immobilized, is characterized in that it has, on the surface thereof, a physiologically active substance that has been immobilized thereon via a hydrophilic polymer layer formed with hydrophilic polymers, and Compound S having a mean molecular weight between 350 and 5,000,000 and having a residue capable of forming a hydrogen bond. In addition, the substrate of the present invention is able to suppress deactivation of the physiologically active substance immobilized on the surface thereof, because it has Compound S.

The substrate of the present invention can be used as a biosensor, for example. The biosensor of the present invention has as broad a meaning as possible, and the term biosensor is used herein to mean a sensor, which converts an interaction between biomolecules into a signal such as an electric signal, so as to measure or detect a target substance. The conventional biosensor is comprised of a receptor site for recognizing a chemical substance as a detection target and a transducer site for converting a physical change or chemical change generated at the site into an electric signal. In a living body, there exist substances having an affinity with each other, such as enzyme/substrate, enzyme/coenzyme, antigen/antibody, or hormone/receptor. The biosensor operates on the principle that a substance having an affinity with another substance, as described above, is immobilized on a substrate to be used as a molecule-recognizing substance, so that the corresponding substance can be selectively measured.

The substrate of the present invention is preferably a metal film which was placed on metal or carrier. A metal constituting the metal surface or metal film is not particularly limited, as long as surface plasmon resonance is generated when the metal is used for a surface plasmon resonance biosensor. Examples of a preferred metal may include free-electron metals such as gold, silver, copper, aluminum or platinum. Of these, gold is particularly preferable. These metals can be used singly or in combination. Moreover, considering adherability to the above carrier, an interstitial layer consisting of chrome or the like may be provided between the carrier and a metal layer.

The film thickness of a metal film is not limited. When the metal film is used for a surface plasmon resonance biosensor, the thickness is preferably between 0.1 nm and 500 nm, and particularly preferably between 1 nm and 200 nm. If the thickness exceeds 500 nm, the surface plasmon phenomenon of a medium cannot be sufficiently detected. Moreover, when an interstitial layer consisting of chrome or the like is provided, the thickness of the interstitial layer is preferably between 0.1 nm and 10 nm.

Formation of a metal film may be carried out by common methods, and examples of such a method may include sputtering method, evaporation method, ion plating method, electroplating method, and nonelectrolytic plating method.

When a substrate of the present invention is used for a surface plasmon resonance biosensor, examples of a carrier may include, generally, optical glasses such as BK7, and synthetic resins. More specifically, materials transparent to laser beams, such as polymethyl methacrylate, polyethylene terephthalate, polycarbonate or a cycloolefin polymer, can be used. For such a substrate, materials that are not anisotropic with regard to polarized light and have excellent workability are preferably used. The description "placed on a carrier" is used herein to mean a case where a metal film is placed on a carrier such that it directly comes into contact with the carrier, as well as a case where a metal film is placed via another layer without directly coming into contact with the carrier. Preferably, the refractive index of a material for the carrier is between 1.4 and 1.7. When an affinity between a physiologically active substance in aqueous solution and a compound is measured by using surface plasmon resonance, a dark line by resonance can not be obtained as reflected light if the refractive index of the substrate is low. Also, incidence angle and reflection angle of light source to the substrate must be low if the refractive index is high, and it may make it difficult to construct the device.

The aforementioned substrate is immobilized on the dielectric block of a measurement unit and is unified therewith to construct a measurement chip. This measurement chip may be exchangeably formed. An example will be given below.

Figure 1 is an exploded perspective view of a sensor unit 10 used in a measurement that utilizes SPR. The sensor unit 10 is composed of a total internal reflection prism (optical block) 20 that is a transparent dielectric body and a flow channel member 30 equipped on the total internal reflection prism 20. The flow channel member 30 has two types of flow channels, namely, a first flow channel 31 located on the back side of the figure and a second flow channel 32 located on the front side of the figure. When the sensor unit 10 is used in measurement, the two types of flow channels 31 and 32 are used in combination to measure a single sample. However, the details will be described later. In the flow channel member 30, six flow channels 31 and six flow channels 32 are established in the longitudinal direction, so that six samples can be measured in a single sensor unit 10. It is to be noted that the number of either the flow channel 31 or 32 is not limited to six, but that it may be 5 or less, or 7 or more.

The total internal reflection prism 20 is composed of a prism main body 21 formed in a long trapezoidal shape, a gripper 22 established at one end of the prism main body 21, and a projecting portion 23 established at the other end of the prism main body 21. This total internal reflection prism 20 is molded by extrusion molding, for example. The prism main body 21, the gripper 22, and the projecting portion 23 are integrally molded.

The prism main body 21 has a substantially trapezoidal longitudinal section wherein the lower base is longer than the upper base. Light irradiated from the lateral side of the bottom is gathered to an upper surface 21 a. A metal film (thin film layer) 25 for exciting SPR is established on the upper surface 21 a of the prism main body 21. The shape of the metal film 25 is rectangular such that it faces the flow channels 31 and 32 of the flow channel member 30. The metal film 25 is molded by an evaporation method, for example. The Metal film 25 is made of gold, silver, or the like, and the thickness thereof is 50 nm, for example. The thickness of the metal film 25 is selected as appropriate, depending on the material of the metal film 25, the wavelength of light irradiated during the measurement, etc.

On the metal film 25, a polymer layer 26 is established. The polymer layer 26 has a binding group for immobilizing a physiologically active substance. A physiologically active substance is immobilized on the metal film 25 via the polymer layer 26.

The metal film in the present invention is coated with a self-assembled membrane-forming molecule (hereinafter referred to as a "step of coating with a self-assembled membrane-forming molecule," as appropriate). Thereafter, a hydrophilic polymer that contains an actively esterified carboxyl group (hereinafter referred to as a "hydrophilic polymer-activating step") is allowed to react with the aforementioned organic layer, so that the hydrophilic polymer capable of immobilizing a physiologically active substance can be established on the substrate (hereinafter referred to as a "hydrophilic polymer-establishing step," as appropriate). Moreover, in the present invention, a physiologically active substance is immobilized on the thus obtained hydrophilic polymer on the substrate (hereinafter referred to as a "physiologically active substance-immobilizing step," as appropriate), and a compound having a residue capable of forming a hydrogen bond is then added thereto (hereinafter referred to as a "stabilizer-adding step," as appropriate), so as to obtain a substrate having excellent preservation stability, on which a physiologically active substance has been immobilized.

### <Step of coating with self-assembled membrane-forming molecule>

In the present invention, the self-assembled membrane-forming molecule has a role for connecting a metal film with a hydrophilic polymer. Hereafter, self-assembled membranes (SAMs) having self-assembled membrane-forming molecules will be described.

A method for coating a metal film with the use of a self-assembled membrane (SAMs) has been actively developed by Professor Whitesides et al. (Harvard University). Details of the method are reported in, for example, Chemical Review, 105, 1103-1169 (2005). When gold is used as a metal, an orientational self-assembled monomolecular film is formed with the use of an alkanethiol derivative (where n represents an integer from 2 to 10) represented by the following formula A-1 based on the van der Waals force between an Au-S bond and an alkyl chain. A self-assembled membrane is formed by a very simple method, wherein a gold substrate is immersed in a solution of an alkanethiol derivative. In the present invention, it is preferred that the compound has an amino group at its terminal. A self-assembled membrane is formed with the use of a compound (represented by the following formula A-1 where X is NH₂) so that it becomes possible to coat a gold surface with an organic layer comprising an amino group:

**HS(CH₂)ₙX** **A-1**

An alkanethiol having an amino group at the end may be a compound comprising a thiol group and an amino group linked via an alkyl chain (formula A-2) (in the formula A-2, n represents an integer of 3 to 20), or may be a compound obtained by reaction between alkanethiol having a carboxyl group at the end (formula A-3 or A-4) (in the formula A-3, n represents an integer of 3 to 20, and in the formula A-4, n each independently represents an integer of 1 to 20) and a large excess of hydrazide or diamine. The reaction between alkanethiol having a carboxyl group at the end and a large excess of hydrazide or diamine may be performed in a solution state. Alternatively, the alkanethiol having a carboxyl group at the end may be bound to the substrate surface and then reacted with a large excess of hydrazide or diamine.

**HS(CH₂)ₙNH₂** **A-2**

**HS(CH₂)ₙCOOH** **A-3**

**HS(CH₂)ₙ(OCH₂CH₂)ₙOCH₂COOH** **A-4**

The repeating number of alkyl group of the formulas A-2 to A-4 is preferably 3 to 20, more preferably 3 to 16, and most preferably 4 to 8. If the alkyl chain is short, formation of self-assembled membrane becomes difficult, and if the alkyl chain is long, water solubility decreases and the handling becomes difficult.

Any compound may be used as the diamine used in the present invention. An aqueous diamine is preferable for use in the biosensor surface. Specific examples of the aqueous diamine may include aliphatic diamine such as ethylenediamine, tetraethylenediamine, octamethylenediamine, decamethylenediamine, piperazine, triethylenediamine, diethylenetriamine, triethylenetetraamine, dihexamethylenetriamine, and 1.4-diaminocyclohexane, and aromatic diamine such as paraphenylenediamine, metaphenylenediamine, paraxylylenediamine, metaxylylenediamine, 4,4'-diaminobiphenyl, 4,4'-diaminodiphenylmethane, 4,4'-diaminodiphenylketone, and 4,4'-diaminodiphenylsulfonic acid. From the viewpoint of increasing the hydrophilicity of the biosensor surface, a compound comprising two amino groups linked via an ethylene glycol unit (formula A-5) may also be used. The diamine used in the present invention is preferably ethylenediamine or the compound represented by the formula A-5 (in the formula A-5, n and m each independently represent an integer of 1 to 20), more preferably ethylenediamine or 1,2-bis(aminoethoxy)ethane (represented by the formula A-5 wherein n=2 and m=1).

**H₂N(CH₂)ₙ(OCH₂CH₂)ₘO(CH₂)ₙNH₂** **A-5**

The alkanethiol having an amino group may form a self-assembled membrane by itself or may form a self-assembled membrane by mixing it with another alkanethiol. It is preferred for use in the biosensor surface that a compound capable of suppressing the nonspecific adsorption of a physiologically active substance should be used as the another alkanethiol. The aforementioned Professor Whitesides et al. have investigated in detail self-assembled membrane capable of suppressing the nonspecific adsorption of a physiologically active substance and have reported that a self-assembled membrane formed from alkanethiol having a hydrophilic group is effective for suppressing nonspecific adsorption (Langmuir, 17, 2841-2850, 5605-5620, and 6336-6343 (2001)). In the present invention, any of compounds described in the aforementioned papers may be used preferably as the alkanethiol that forms a mixed monolayer with an alkanethiol having an amino group. In terms of excellent ability to suppress nonspecific adsorption and ease of acquisition, it is preferred that alkanethiol having a hydroxyl group (formula A-6) or alkanethiol having an ethylene glycol unit (formula A-7) (in the formula A-6, n represents an integer of 3 to 20, and in the formula A-7, n and m each independently represent an integer of 1 to 20) should be used as the alkanethiol that forms a mixed monolayer with an alkanethiol having an amino group.

**HS(CH₂)ₙOH** **A-6**

**HS(CH₂)ₙ(OCH₂CH₂)ₘOH** **A-7**

When alkane thiol having an amino group is mixed with another alkane thiol to form a self-assembled membrane, the repeating number of alkyl group of the formulas A-2 to A-4 is preferably 4 to 20, more preferably 4 to 16, and most preferably 4 to 10. Further, the repeating number of alkyl group of the formulas A-6 and A-7 is preferably 3 to 16, more preferably 3 to 12, and most preferably 3 to 8.

In the present invention, it is possible to mix alkanethiol having an amino group and alkanethiol having a hydrophilic group at an arbitrary ratio. However, when the content of alkanethiol having an amino group is low, the amount of actively esterified carboxyl group-containing polymer to be bound decreases. When the content of alkanethiol having a hydrophilic group is low, the capacity for suppression of nonspecific adsorption is reduced. Thus, the mixing ratio of alkanethiol having an amino group to alkanethiol having a hydrophilic group is preferably 1:1 to 1:1,000,000, more preferably 1:1 to 1:1,000, and further preferably 1:1 to 1:10. In view of reduction of steric hindrance upon a reaction with an actively esterified carboxyl group-containing polymer, the molecular length of alkanethiol having an amino group is preferably longer than that of alkanethiol having a hydrophilic group.

As alkanethiol used for the present invention, compounds synthesized based on Abstract, Curr. Org. Chem., 8, 1763-1797 (2004) (Professor Grzybowski, Northwestern University) and references cited therein or a commercially available compound may be used. It is possible to purchase such compounds from Dojindo Laboratories, Aldrich, SensoPath Technologies, Frontier Scientific Inc., and the like. In the present invention, disulfide compounds that are oxidation products of alkanethiol can be used in the same manner as alkanethiol.

### <Hydrophilic polymer-activating step >

The polymer containing a carboxyl group that is used in the present invention includes a synthetic polymer containing a carboxyl group and polysaccharide containing a carboxyl group. Examples of a synthetic polymer containing a carboxyl group include polyacrylic acid, polymethacrylic acid, and copolymers of such acids, including methacrylic acid copolymer, acrylic acid copolymer, itaconic acid copolymer, crotonic acid copolymer, maleic acid copolymer, partially esterified maleic acid copolymer, and a polymer containing a hydroxyl group to which acid anhydride is added described in JP Patent Publication (Kokai) No. 59-53836 A (1984) (page 3, lines 20 to page 6, line 49 of the specification) and JP Patent Publication (Kokai) No. 59-71048 A (1984) (page 3, lines 41 to page 7, line 54 of the specification). A polysaccharide containing a carboxyl group may be extracts from natural plants, microbial fermentation products, enzymatically synthesized products, or chemically synthesized products. Specific examples thereof include hyaluronic acid, chondroitin sulfate, heparin, dermatan sulfate, carboxymethyl cellulose, carboxyethyl cellulose, cellouronic acid, carboxymethyl chitin, carboxymethyl dextran, and carboxymethyl starch. As such polysaccharide containing a carboxyl group, it is possible to use a commercially available compound. Specific examples thereof include carboxymethyldextrans such as CMD, CMD-L, and CMD-D40 (Meito Sangyo Co., Ltd.), sodium carboxymethyl cellulose (Wako Pure Chemical Industries, Ltd.), and sodium alginate (Wako Pure Chemical Industries, Ltd.).

A polymer containing a carboxyl group is preferably a polysaccharide containing a carboxyl group and more preferably carboxymethyl dextran.

The molecular weight of the polymer containing a carboxyl group used in the present invention is not particularly limited. However, the average molecular weight is preferably 1,000 to 5,000,000, more preferably 10,000 to 2,000,000. When the average molecular weight is below the aforementioned scope, the amount of physiologically active substance immobilized becomes small. When the average molecular weight exceeds the aforementioned scope, it is difficult to handle the polymer due to a high solution viscosity.

A known technique can be preferably used as a method for activating polymers containing carboxyl groups. Preferred examples of such method include: a method that involves activating carboxyl groups using 1-(3-Dimethylaminopropyl)-3 ethylcarbodiimide (EDC) (water-soluble carbodiimide) alone, or using EDC and N-Hydroxysuccinimide (NHS). It becomes possible to produce the substrate of the present invention by causing a polymer containing a carboxyl group that has been activated by these techniques to react with a substrate having an amino group.

Moreover, another method for activation of a polymer containing a carboxyl groups is a method that uses a nitrogen-containing compound. Specifically, a nitrogen-containing compound represented by the following formula (Ia) or (Ib) [wherein R₁ and R₂ mutually independently denote a carbonyl group, a carbon atom, or a nitrogen atom, which may have a substituent, R₁ and R₂ may form 5- to 6-membered rings via binding, "A" denotes a carbon atom or a phosphorus atom, which has a substituent, "M" denotes an (n-1)-valent element, and "X" denotes a halogen atom] can also be used.

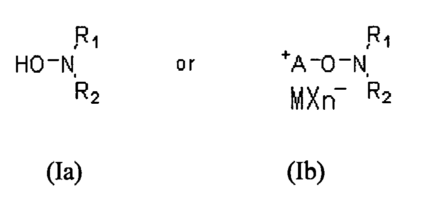

R₁ and R₂ mutually independently denote a carbonyl group, a carbon atom, or a nitrogen atom, which may have a substituent, and preferably R₁ and R₂ form 5- to 6-membered rings via binding. Particularly preferably, hydroxysuccinic acid, hydroxyphthalic acid, 1-hydroxybenzotriazole, 3,4-dihydroxy-3-hydroxy-4-oxo-1,2,3-benzotriazine, and a derivative thereof are provided.

Further preferably, a nitrogen-containing compound represented by the following compound can also be used.

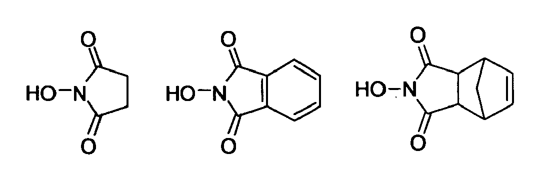

More preferably, a compound represented by the following formula (II) [wherein "Y" and "Z" mutually independently denote CH or a nitrogen atom] can also be used as a nitrogen-containing compound.

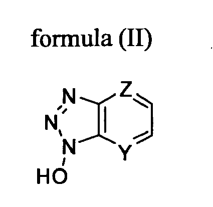

Specifically, the following compounds can be used, for example.

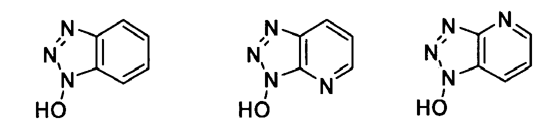

Further preferably, the following compound can also be used as a nitrogen-containing compound.

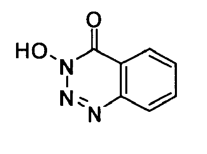

Further preferably, a compound represented by the following formula (III) [wherein "A" denotes a carbon atom or a phosphorus atom, which has a substituent, "Y" and "Z" mutually independently denote CH or a nitrogen atom, "M" denotes a (n-1)-valent element, and X denotes a halogen atom] can also be used as a nitrogen-containing compound.

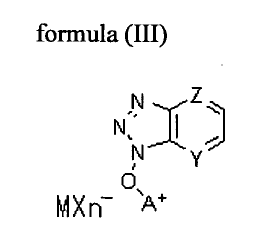

A substituent of a carbon atom or a phosphorus atom denoted by "A" is preferably an amino group having a substituent. Furthermore, a dialkylamino group such as a dimethylamino group or a pyrrolidino group is preferable. Examples of an (n-1)-valent element denoted by "M" include a phosphorus atom, a boron atom, and an arsenic atom. A preferable example of such (n-1)-valent element is a phosphorus atom. Examples of a halogen atom denoted by "X" include a fluorine atom, a chlorine atom, a bromine atom, and an iodine atom. A preferable example of such halogen atom is a fluorine atom.

Furthermore, specific examples of such nitrogen-containing compound represented by formula (III) include the following compounds, for example.

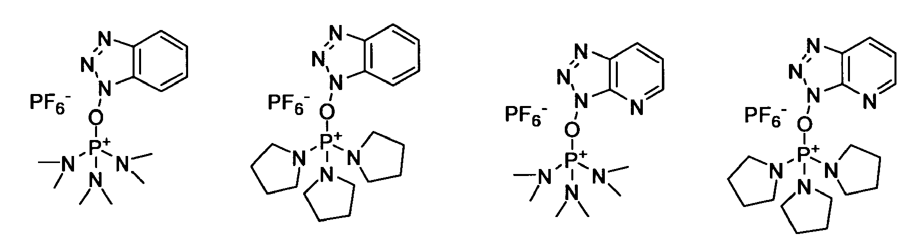

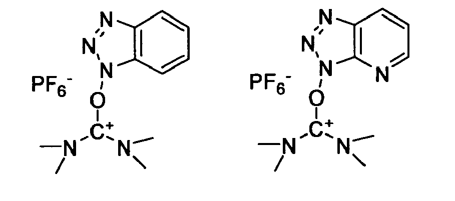

Further preferably, a compound represented by the following formula (IV) [wherein "A" denotes a carbon atom or a phosphorus atom, which has a substituent, "M" denotes an (n-1)-valent element, and "X" denotes a halogen atom] can also be used as a nitrogen-containing compound. 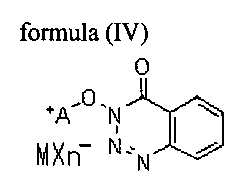 Specifically, the following compound can be used, for example.

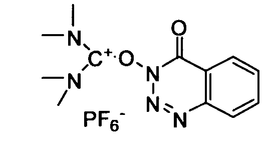

The mixing ratio of the above nitrogen compound as an activating agent may be any amount which is generally used in this purpose of use. In view of immobilizing the polymer sufficiently, it is preferred that the mixing molar ratio of the nitrogen compound to a functional group (for example, carboxyl group) in the polymer layer 26 is 1 x 10⁻⁷ to 1.

Moreover, as a method for activating a polymer containing carboxyl group, the use of a phenol derivative having an electron-withdrawing group is also preferable. Furthermore, the σ value of the electron-withdrawing group is preferably 0.3 or higher. Specifically, the following compounds or the like can also be used.

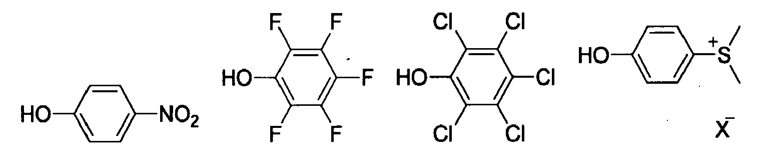

The mixing ratio of the above phenol compound as an activating agent may be any amount which is generally used in this purpose of use. In view of immobilizing the polymer sufficiently, it is preferred that the mixing molar ratio of the phenol compound to a functional group (for example, carboxyl group) in the polymer layer 26 is 1 x 10⁻⁷ to 1.

Furthermore, a carbodiimide derivative can further be used separately for such method for activating a polymer containing carboxyl group in combination with the above compounds. Preferably, a water-soluble carbodiimide derivative can be used in combination with such compounds. Further preferably, the following compound (1-Ethyl-3-(3-dimethylaminopropyl)carbodiimide, hydrochloride) can be used in combination with such compounds.

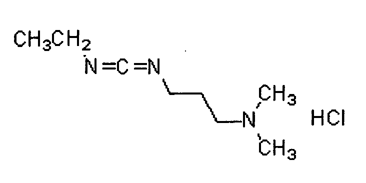

The above carbodiimide derivative and nitrogen-containing compound or phenol derivative can be used not only in such manner, but also independently, if desired. Preferably, a carbodiimide derivative and a nitrogen-containing compound are used in combination.

Furthermore, the morpholine derivative can be used can also be used in such method for activating carboxyl groups. The compound can be used independently and can also be used in combination with a carbodiimide derivative, a nitrogen-containing compound, and/or a phenol derivative. The compound of the following formula (VII) can be used as a morpholine derivative. The mixing ratio of the morpholone derivative as an activating agent may be any amount which is generally used in this purpose of use. In view of immobilizing the polymer sufficiently, it is preferred that the mixing molar ratio of the morpholine compound to a functional group (for example, carboxyl group) in the polymer layer 26 is 1 x 10⁻⁴ to 1.

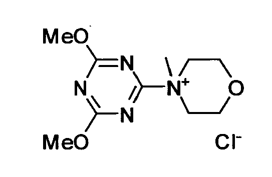

### <Hydrophilic polymer-establishing step>

In the present invention, a polymer containing an actively esterified carboxyl group, which is in the form of a solution, may be allowed to react with a substrate. Otherwise, it may also be allowed to react therewith in a state where a thin film has been formed on a substrate by methods such as spin coating. The reaction is preferably carried out in a state where a thin film has been formed is preferable.

As stated above, the polymer containing an actively esterified carboxyl group in the present invention may be preferably allowed to react with a substrate in the state of a thin film. As a method for forming a thin film on a substrate, known methods can be used. Specific examples of such methods that can be used include an extrusion coating method, a curtain coating method, a casting method, a screen printing method, a spin coating method, a spray coating method, a slide bead coating method, a slit and spin method, a slit coating method, a dye coating method, a dip coating method, a knife coating method, a blade coating method, a flow coating method, a roll coating method, a wire-bar coating method, and a transfer printing method. These methods for forming a thin film are described in "Progress in Coating Technology (Coating Gijutsu no Shinpo)" written by Yuji Harazaki, Sogo Gijutsu Center (1988); "Coating Technology (Coating Gijutsu)" Technical Information Institute Co., Ltd. (1999); "Aqueous Coating Technology (Suisei Coating no Gijutsu)" CMC (2001); "Evolving Organic Thin Film: Edition for Deposition (Shinka-suru Organic Thin Film: Seimaku hen)" Sumibe Techno Research Co., Ltd. (2004); "Polymer Surface Processing Technology (Polymer Hyomen Kako Gaku)" written by Akira Iwamori, Gihodo Shuppan Co., Ltd. (2005); and the like. As the method for forming a thin film on a substrate of the present invention, a spray coating method or a spin coating method is preferable. Further, a spin coating method is more preferable. This is because it allows a coating film having a controlled film thickness to be readily produced.

The spray coating method is a method wherein a substrate is moved with an ultra-atomized polymer solution sprayed onto the substrate to thereby uniformly coat the polymer solution onto the substrate. When the trigger of a spray gun is pulled, an air valve and a needle valve are simultaneously opened. The polymer solution is ejected in the form of a fine mist from a nozzle, and this polymer solution in the form of a fine mist is further ultra-atomized by air ejected from an air cap located at the end of the nozzle. A thickness-controlled polymer film is easily produced by forming the coating film of the ultra-atomized polymer solution on the substrate surface, followed by the evaporation of the solvent. The thickness of the polymer thin film can be controlled on the basis of the concentration of the polymer solution, the moving speed of the substrate, and so on.

The spin coating method is a method wherein a polymer solution is added dropwise onto a substrate placed horizontally, which is then spun at a high speed to thereby uniformly coat the polymer solution onto the whole surface of the substrate through a centrifugal force. A thickness-controlled polymer film is easily produced with the scattering of the polymer solution through a centrifugal force and the evaporation of the solvent. The thickness of the polymer thin film can be controlled on the basis of the revolution speed, the concentration of the polymer solution, the vapor pressure of the solvent, and so on. In the present invention, the revolution speed during spin coating is not particularly limited. If the revolution speed is too small, the solution remains on the substrate. If the revolution speed is too large, an available apparatus is restricted. Hence, in the present study, the revolution speed during spin coating is preferably 500 rpm to 10,000 rpm, more preferably 1,000 rpm to 7,000 rpm.

### <Physiologically active substance-immobilizing step>

A polymer containing a carboxyl group which was established by the aforementioned method, is activated by a known method using water-soluble carbodiimide, 1-(3-dimethylaminopropyl)-3 ethylcarbodiimide (EDC) alone, or using EDC and N-hydroxysuccinimide (NHS), for example, so that it is able to immobilize a physiologically active substance having an amino group. As a method of activating carboxylic acid, the method described in Japanese Patent Application No. 2004-238396 (JP Patent Publication (Kokai) No.2006-58071A), paragraphs [0011] to [0022] (that is, a method of activating a carboxyl group existing on the surface of a substrate using any compound selected from a uronium salt, a phosphonium salt, and a triazine derivative, which have a specific structure, so as to form a carboxylic amide group) and the method described in Japanese Patent Application No. 2004-275012 (JP Patent Publication (Kokai) No.2006-90781A), paragraphs [0011] to [0019] (that is, a method, which comprises activating a carboxyl group existing on the surface of a substrate using a carbodiimide derivative or a salt thereof, converting the resultant to an ester using any compound selected from a nitrogen-containing hetero aromatic compound having a hydroxyl group, a phenol derivative having an electron attracting group, and an aromatic compound having a thiol group, and allowing the ester to react with amine, so as to form a carboxylic amide group) can preferably be used.

It is to be noted that the aforementioned uronium salt, phosphonium salt, and triazine derivative, which have a specific structure, described in Japanese Patent Application No. 2004-238396(JP Patent Publication (Kokai) No.2006-58071A), mean the uronium salt represented by the following formula 1, the phosphonium salt represented by the following formula 2, and the triazine derivative represented by the following formula 3, respectively. 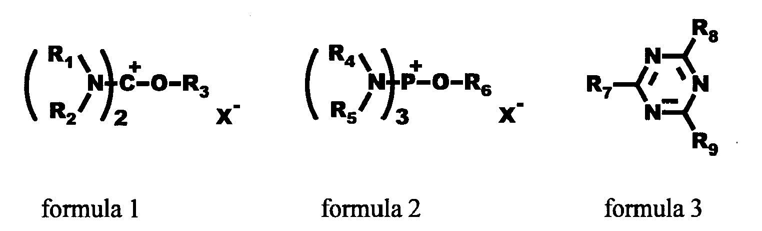 (in formula 1, each of R₁ and R₂ independently represents an alkyl group containing 1 to 6 carbon atoms, or R₁ and R₂ together form an alkylene group containing 2 to 6 carbon atoms, which forms a ring together with an N atom, R₃ represents an aromatic ring group containing 6 to 20 carbon atoms, or a hetero ring group containing at least one heteroatom, and X⁻ represents an anion; in formula 2, each of R₄ and R₅ independently represents an alkyl group containing 1 to 6 carbon atoms, or R₄ and R₅ together form an alkylene group containing 2 to 6 carbon atoms, which forms a ring together with an N atom, R₆ represents an aromatic ring group containing 6 to 20 carbon atoms, or a hetero ring group containing at least one heteroatom, and X⁻ represents an anion; and in formula 3, R₇ represents an onium group, and each of R₈ and R₉ independently represents an electron donating group.)

A physiologically active substance immobilized on the substrate of the present invention is not particularly limited, as long as it interacts with a measurement target. Examples of such a substance may include an immune protein, an enzyme, a microorganism, nucleic acid, a low molecular weight organic compound, a nonimmune protein, an immunoglobulin-binding protein, a sugar-binding protein, a sugar chain recognizing sugar, fatty acid or fatty acid ester, and polypeptide or oligopeptide having a ligand-binding ability.

Examples of an immune protein may include an antibody whose antigen is a measurement target, and a hapten. Examples of such an antibody may include various immunoglobulins such as IgG, IgM, IgA, IgE or IgD. More specifically, when a measurement target is human serum albumin, an anti-human serum albumin antibody can be used as an antibody. When an antigen is an agricultural chemical, pesticide, methicillin-resistant *Staphylococcus aureus,* antibiotic, narcotic drug, cocaine, heroin, crack or the like, there can be used, for example, an anti-atrazine antibody, anti-kanamycin antibody, anti-metamphetamine antibody, or antibodies against O antigens 26, 86, 55, 111 and 157 among enteropathogenic *Escherichia coli.*

An enzyme used as a physiologically active substance herein is not particularly limited, as long as it exhibits an activity to a measurement target or substance metabolized from the measurement target. Various enzymes such as oxidoreductase, hydrolase, isomerase, lyase or synthetase can be used. More specifically, when a measurement target is glucose, glucose oxidase is used, and when a measurement target is cholesterol, cholesterol oxidase is used. Moreover, when a measurement target is an agricultural chemical, pesticide, methicillin-resistant *Staphylococcus aureus,* antibiotic, narcotic drug, cocaine, heroin, crack or the like, enzymes such as acetylcholine esterase, catecholamine esterase, noradrenalin esterase or dopamine esterase, which show a specific reaction with a substance metabolized from the above measurement target, can be used.

A microorganism used as a physiologically active substance herein is not particularly limited, and various microorganisms such as *Escherichia coli* can be used.

As nucleic acid, those complementarily hybridizing with nucleic acid as a measurement target can be used. Either DNA (including cDNA) or RNA can be used as nucleic acid. The type of DNA is not particularly limited, and any of native DNA, recombinant DNA produced by gene recombination and chemically synthesized DNA may be used.

As a low molecular weight organic compound, any given compound that can be synthesized by a common method of synthesizing an organic compound can be used.

A nonimmune protein used herein is not particularly limited, and examples of such a nonimmune protein may include avidin (streptoavidin), biotin, and a receptor. Examples of an immunoglobulin-binding protein used herein may include protein A, protein G, and a rheumatoid factor (RF). As a sugar-binding protein, for example, lectin is used. Examples of fatty acid or fatty acid ester may include stearic acid, arachidic acid, behenic acid, ethyl stearate, ethyl arachidate, and ethyl behenate.

As a physiologically active substance to be immobilized on a substrate in the present invention, a ligand that forms a bond with a tag molecule (a molecule that forms a specific affinity with a specific ligand) at Kd (dissociation constant) of 10⁻¹² M or less is preferable. Specifically, protein A, protein G, avidins, calmodulin, and an antibody are preferable. A ligand that forms a bond with such a tag molecule at Kd of 10⁻¹² M or less is used as a binding group, and it becomes possible to immobilize a specific physiologically active substance modified with the tag molecule on a substrate, while suppressing a decrease in the activity of the aforementioned specific physiologically active substance. Examples of a combination of a tag molecule with a ligand include a biotin/a biotin-binding protein, a digoxigenin/a digoxigenin antibody, and a D-Ala-D-Ala derivative/a vancomycin trimer derivative described in SCIENCE, 280, 708-711 (1998). Specific examples of a biotin-binding protein include avidins (avidin, streptavidin, NeutrAvidin, or a modified compound thereof, etc.) Such avidins are particularly preferable in terms of stability on a substrate and a low Kd value.

In the present invention, a solution that contains a physiologically active substance capable of forming a covalent bond with a molecule that constitutes a layer on which the aforementioned physiologically active substance is immobilized is applied onto the surface of a metal substrate having the physiologically active substance-immobilized layer, and the solution is then dried, so as to form a uniform film on the surface of the substrate. At that time, the physiologically active substance covalently binds to an activated carboxyl group in a hydrophilic polymer, so that it can be immobilized on the surface of the metal substrate.

With regard to the concentration of a solution (an applied solution) that contains physiologically active substance, it is preferable that the concentration of physiologically active substance immobilized on a substrate surface be high. The aforementioned concentration is preferably between 0.1 mg/ml and 10 mg/ml, and more preferably between 1 mg/ml and 10 mg/ml, although it depends on the type of physiologically active substance.

In the process of drying such a solution that contains physiologically active substance, the physiologically active substance tend to be precipitated from the peripheral portion of the applied solution, or from a portion in which the liquid remains immediately before the applied solution is dried. Thereby, the quantities of physiologically active substance immobilized on the substrate surface vary. This is not preferable. In order to uniform the quantities of physiologically active substance immobilized on the substrate surface, it is preferable that the viscosity of the applied solution be set at high, so far as it does not inhibit the binding of the physiologically active substance to the substrate surface. By setting the viscosity of the applied solution at high, the movement of the physiologically active substance contained in the applied solution in the horizontal direction towards the substrate surface can be suppressed during the drying process. As a result, variations in the quantities of physiologically active substance immobilized can be suppressed. The viscosity of the applied solution is preferably maintained at 0.9 cP or more during the drying process.

An increase in the drying rate of the solution that contains the physiologically active substance (coating solution) is also effective for suppressing variation in the amount of the physiologically active substance immobilized. The drying rate is increased, and the drying process is sufficiently rapidly completed with respect to the movement of the physiologically active substance in the horizontal direction. Thereby, the drying process is completed before the physiologically active substance substantially moves, so that the variation can be suppressed. When such a coating solution contains water, a high drying rate can be obtained by drying the coating solution in an environment wherein a difference between a dry-bulb temperature and a wet-bulb temperature is great. The coating solution is dried in an environment wherein a temperature difference between the dry-bulb temperature and the wet-bulb temperature is preferably 7°C or greater, and more preferably 10°C or greater. In addition, considering the production process, the drying time is preferably 10 minutes or shorter, more preferably 5 minutes or shorter, and particularly preferably 1 minute or shorter.

An example of a method of applying a solution that contains physiologically active substance is a method particularly using a dispenser that quantitatively discharges a solution to be applied. The discharge port of the dispenser is moved on a substrate at a certain speed at certain intervals, so that the solution can be uniformly applied at any given sites on the substrate. When the solution is applied using a dispenser, the interval between the substrate and the discharge port is extremely narrowed, and the thickness of the applied solution is reduced, so that the thickness of physiologically active substance can be uniformed. Further, the drying speed can also be increased. Thus, the use of such a dispenser is preferable. Another preferred method of applying a solution that contains physiologically active substance is spin coating. This method is particularly preferably applied when the thickness of the applied film is reduced. In this method, since the drying process is carried out after a solution having a uniformed thickness has been formed, it is preferable to prevent evaporation of the solution during rotation of a spin coater. If a method of placing a substrate in a hermetically sealed vessel or the like during rotation is applied to maintain the concentration of a solvent existing around the substrate at high, the drying speed can be controlled before and after formation of a thin film during rotation. Thus, this method is particularly preferable.

When the interaction of physiologically active substance with test substances is detected, variations in the quantities of physiologically active substance immobilized on the sensor surface cause an error in quantitative and kinetic evaluation of such an interaction. In order to keep such an error to a minimum, the quantities of physiologically active substance immobilized are preferably uniformed. A CV value (coefficient variation) (standard deviation/mean value), which indicates variations in the quantities of physiologically active substance immobilized on the surface of a substrate used in detection of an interaction, is preferably 15% or less, and more preferably 10% or less. Such a CV value can be calculated based on the quantities of physiologically active substance immobilized on at least two sites, preferably 10 or more sites, and more preferably 100 or more sites on the substrate surface. The amount of immobilized physiologically active substance is preferably 1ng/mm² to 50ng/mm².

Uniformity can be evaluated by quantifying the quantities of substances existing on a sensor substrate before and after immobilization of physiologically active substance. However, such uniformity can also be evaluated by fluorescently-labeling substances that have been known to bind to physiologically active substance, immobilizing such fluorescently-labeled substances on a sensor substrate, and then measuring fluorescence intensity using a fluorescence microscope or the like. Moreover, it is also possible to quantify physiologically active substance using an SPR imager, an ellipsometer, TOF-SIMS, an ATR-IR apparatus, etc.

### <Stabilizer-adding step>

Generally, physiologically active substance such as protein maintain its three-dimensional structure by coordination of water molecules in a solution, but when it is dried, physiologically active substance cannot maintain its three-dimensional structure and is denatured. Further, physiologically active substance is contained in a hydrophilic polymer compound on a surface of substrate, physiologically active substance aggregate by drying, and aggregates are produced. The compound S having a residue capable of forming hydrogen bond which may be used in the present invention can be used for the purpose of suppressing denature of physiologically active substance by maintaining the three-dimensional structure in place of water or suppressing the aggregation by steric effect by covering the physiologically active substance.

In the present invention, the Compound S having a residue capable of forming hydrogen bond is preferably added as a aqueous solution to a layer on substrate where physiologically active substance was immobilized. Compound S can be added by coating a mixed solution of Compound S and physiologically active substance on a surface of substrate, or by immobilizing physiologically active substances on a surface of substrate and then over-coating the Compound S. When a mixed solution of Compound S and physiologically active substance is coated, fluctuation of the amount of immobilized physiologically active substances can be reduced. Preferably, an aqueous solution of Compound S can be added to substrate in a state of thin film. A method for forming thin film on substrate may be any known method. Examples thereof include extrusion coating, curtain coating, casting, screen printing, spin coating, spray coating, slidebead coating, slit and spin coating, slit coating, die coating, dip coating, knife coating, blade coating, flow coating, roll coating, wire-bar coating, and transferring printing. In the present invention, spray coating or spin coating is preferably used, and spin coating is more preferably used as a method for forming a thin film on substrate, since a coated film having a controlled film thickness can be easily prepared.

The concentration of the applied solution of compound S is not particularly limited, as long as it does not cause a problem regarding permeation into a layer that contains physiologically active substances. The aforementioned concentration is preferably between 0.1% by weight and 5% by weight. In addition, in terms of applicability and regulation of pH, a surfactant, a buffer, an organic solvent, a salt may also be added to the applied solution.

The compound S having a residue capable of forming hydrogen bond is preferably a compound which is non-volatile under normal pressure at normal temperature. The average molecular weight of the compound is preferably 350 to 5,000,000, more preferably 1,200 to 2,000,000, most preferably 1,200 to 70,000. The compound S having a hydroxyl group in molecule is preferably saccharide. The saccharide may be monosaccharide or .polysaccharide. In case of n-saccharide, n is preferably 4 to 1,200, and n is more preferably 20 to 600.

If the mean molecular weight of compound S is too low, the compound is crystallized on the surface of a substrate. This causes disruption of a hydrophilic polymer layer, on which physiologically active substances are immobilized, and disruption of the three-dimensional structure of the physiologically active substances. In contrast, if the mean molecular weight of compound S is too high, it causes problems such that it impairs immobilization of physiologically active substances on a substrate, that a layer that contains physiologically active substances cannot be impregnated with compound S, and that layer separation occurs.

For the purpose of suppressing degradation of physiologically active substances immobilized on a substrate, the aforementioned compound S having a residue capable of forming hydrogen bond preferably has a dextran skeleton or a polyethylene oxide skeleton. The type of a substituent used is not limited, as long as the object of the present invention can be achieved. Moreover, for the purpose of suppressing degradation of physiologically active substances immobilized on a substrate, a nonionic compound having no dissociable groups is preferably used as compound S. Furthermore, the aforementioned compound S having a residue capable of forming a hydrogen bond preferably has high affinity for water molecules. A distribution coefficient LogP value between water and n-octanol is preferably 1 or greater. Such LogP value can be measured by the method described in Japanese Industrial Standard (JIS), Z7260-107 (2000), "Measurement of distribution coefficient (1-octanol/water) - Shaking method," etc.

Specific examples of compound S having a residue capable of forming hydrogen bond include: compounds consisting of two or more types of residues selected from polyalcohols such as polyvinyl alcohol, proteins such as collagen, gelatin, or albumin, polysaccharides such as hyaluronic acid, chitin, chitosan, starch, cellulose, alginic acid, or dextran, polyethers including polyethyleneoxy-polypropylene oxide condensates such as polyethylene glycol, polyethylene oxide, polypropylene glycol, polypropylene oxide, or Pluronic, Tween 20, Tween 40, Tween 60, Tween 80, etc.; and derivatives and polymers of such compounds. Of these, polysaccharides and polyethers are preferable, and polysaccharides are more preferable. Specifically, dextran, cellulose, Tween 20, Tween 40, Tween 60, and Tween 80 are preferably used. It is preferable that such compound S be substantially identical to the basic skeleton of a hydrophilic polymer used in the present invention. The term "basic skeleton" is used herein to mean a ring structure of sugar, for example. Although the type of a functional group or length differs, if such a ring structure is identical, it is considered that the basic skeleton is substantially identical.

With regard to the content of compound S having a residue capable of forming hydrogen bond existing on a substrate, the ratio of the mean molecular weight of the aforementioned compound S to the mean molecular weight of a hydrophilic polymer is preferably between 0.005 and 0.2. If such a ratio is lower than the aforementioned range, compound S is likely to be crystallized. If such a ratio is higher than the aforementioned range, it is difficult for compound S to permeate into a hydrophilic polymer layer. When the aforementioned ratio is set within the aforementioned range, such problems are solved. Thereby, a higher effect of suppressing denature of physiologically active substances and a higher effect of suppressing aggregation can be obtained.

A substrate to which a physiologically active substance is immobilized as described above can be used to detect and/or measure a substance which interacts with the physiologically active substance.

In the present invention, it is preferable to detect and/or measure an interaction between a physiologically active substance immobilized on the substrate and a test substance by a nonelectric chemical method. Examples of a non-electrochemical method may include a surface plasmon resonance (SPR) measurement technique, a quartz crystal microbalance (QCM) measurement technique, and a measurement technique that uses functional surfaces ranging from gold colloid particles to ultra-fine particles.

In a preferred embodiment of the present invention, the substrate of the present invention can be used as a biosensor for surface plasmon resonance which is characterized in that it comprises a metal film placed on a transparent substrate.

A biosensor for surface plasmon resonance is a biosensor used for a surface plasmon resonance biosensor, meaning a member comprising a portion for transmitting and reflecting light emitted from the sensor and a portion for immobilizing a physiologically active substance. It may be fixed to the main body of the sensor or may be detachable.

The surface plasmon resonance phenomenon occurs due to the fact that the intensity of monochromatic light reflected from the border between an optically transparent substance such as glass and a metal thin film layer depends on the refractive index of a sample located on the outgoing side of the metal. Accordingly, the sample can be analyzed by measuring the intensity of reflected monochromatic light.

A device using a system known as the Kretschmann configuration is an example of a surface plasmon measurement device for analyzing the properties of a substance to be measured using a phenomenon whereby a surface plasmon is excited with a lightwave (for example, Japanese Patent Laid-Open No. 6-167443). The surface plasmon measurement device using the above system basically comprises a dielectric block formed in a prism state, a metal film that is formed on a face of the dielectric block and comes into contact with a measured substance such as a sample solution, a light source for generating a light beam, an optical system for allowing the above light beam to enter the dielectric block at various angles so that total reflection conditions can be obtained at the interface between the dielectric block and the metal film, and a light-detecting means for detecting the state of surface plasmon resonance, that is, the state of attenuated total reflection, by measuring the intensity of the light beam totally reflected at the above interface.

In order to achieve various incident angles as described above, a relatively thin light beam may be caused to enter the above interface while changing an incident angle. Otherwise, a relatively thick light beam may be caused to enter the above interface in a state of convergent light or divergent light, so that the light beam contains components that have entered therein at various angles. In the former case, the light beam whose reflection angle changes depending on the change of the incident angle of the entered light beam can be detected with a small photodetector moving in synchronization with the change of the above reflection angle, or it can also be detected with an area sensor extending along the direction in which the reflection angle is changed. In the latter case, the light beam can be detected with an area sensor extending to a direction capable of receiving all the light beams reflected at various reflection angles.

With regard to a surface plasmon measurement device with the above structure, if a light beam is allowed to enter the metal film at a specific incident angle greater than or equal to a total reflection angle, then an evanescent wave having an electric distribution appears in a measured substance that is in contact with the metal film, and a surface plasmon is excited by this evanescent wave at the interface between the metal film and the measured substance. When the wave vector of the evanescent light is the same as that of a surface plasmon and thus their wave numbers match, they are in a resonance state, and light energy transfers to the surface plasmon. Accordingly, the intensity of totally reflected light is sharply decreased at the interface between the dielectric block and the metal film. This decrease in light intensity is generally detected as a dark line by the above light-detecting means. The above resonance takes place only when the incident beam is p-polarized light. Accordingly, it is necessary to set the light beam in advance such that it enters as p-polarized light.

If the wave number of a surface plasmon is determined from an incident angle causing the attenuated total reflection (ATR), that is, an attenuated total reflection angle (θSP), the dielectric constant of a measured substance can be determined. As described in Japanese Patent Laid-Open No. 11-326194, a light-detecting means in the form of an array is considered to be used for the above type of surface plasmon measurement device in order to measure the attenuated total reflection angle (θSP) with high precision and in a large dynamic range. This light-detecting means comprises multiple photo acceptance units that are arranged in a certain direction, that is, a direction in which different photo acceptance units receive the components of light beams that are totally reflected at various reflection angles at the above interface.

In the above case, there is established a differentiating means for differentiating a photodetection signal outputted from each photo acceptance unit in the above array-form light-detecting means with regard to the direction in which the photo acceptance unit is arranged. An attenuated total reflection angle (θSP) is then specified based on the derivative value outputted from the differentiating means, so that properties associated with the refractive index of a measured substance are determined in many cases.

In addition, a leaking mode measurement device described in "Bunko Kenkyu (Spectral Studies)" Vol. 47, No. 1 (1998), pp. 21 to 23 and 26 to 27 has also been known as an example of measurement devices similar to the above-described device using attenuated total reflection (ATR). This leaking mode measurement device basically comprises a dielectric block formed in a prism state, a clad layer that is formed on a face of the dielectric block, a light wave guide layer that is formed on the clad layer and comes into contact with a sample solution, a light source for generating a light beam, an optical system for allowing the above light beam to enter the dielectric block at various angles so that total reflection conditions can be obtained at the interface between the dielectric block and the clad layer, and a light-detecting means for detecting the excitation state of waveguide mode, that is, the state of attenuated total reflection, by measuring the intensity of the light beam totally reflected at the above interface.

In the leaking mode measurement device with the above structure, if a light beam is caused to enter the clad layer via the dielectric block at an incident angle greater than or equal to a total reflection angle, only light having a specific wave number that has entered at a specific incident angle is transmitted in a waveguide mode into the light wave guide layer, after the light beam has penetrated the clad layer. Thus, when the waveguide mode is excited, almost all forms of incident light are taken into the light wave guide layer, and thereby the state of attenuated total reflection occurs, in which the intensity of the totally reflected light is sharply decreased at the above interface. Since the wave number of a waveguide light depends on the refractive index of a measured substance placed on the light wave guide layer, the refractive index of the measurement substance or the properties of the measured substance associated therewith can be analyzed by determining the above specific incident angle causing the attenuated total reflection.

In this leaking mode measurement device also, the above-described array-form light-detecting means can be used to detect the position of a dark line generated in a reflected light due to attenuated total reflection. In addition, the above-described differentiating means can also be applied in combination with the above means.

The above-described surface plasmon measurement device or leaking mode measurement device may be used in random screening to discover a specific substance binding to a desired sensing substance in the field of research for development of new drugs or the like. In this case, a sensing substance is immobilized as the above-described measured substance on the above thin film layer (which is a metal film in the case of a surface plasmon measurement device, and is a clad layer and a light guide wave layer in the case of a leaking mode measurement device), and a sample solution obtained by dissolving various types of test substance in a solvent is added to the sensing substance. Thereafter, the above-described attenuated total reflection angle (θSP) is measured periodically when a certain period of time has elapsed.

If the test substance contained in the sample solution is bound to the sensing substance, the refractive index of the sensing substance is changed by this binding over time. Accordingly, the above attenuated total reflection angle (θSP) is measured periodically after the elapse of a certain time, and it is determined whether or not a change has occurred in the above attenuated total reflection angle (θSP), so that a binding state between the test substance and the sensing substance is measured. Based on the results, it can be determined whether or not the test substance is a specific substance binding to the sensing substance. Examples of such a combination between a specific substance and a sensing substance may include an antigen and an antibody, and an antibody and an antibody. More specifically, a rabbit anti-human IgG antibody is immobilized as a sensing substance on the surface of a thin film layer, and a human IgG antibody is used as a specific substance.

It is to be noted that in order to measure a binding state between a test substance and a sensing substance, it is not always necessary to detect the angle itself of an attenuated total reflection angle (θSP). For example, a sample solution may be added to a sensing substance, and the amount of an attenuated total reflection angle (θSP) changed thereby may be measured, so that the binding state can be measured based on the magnitude by which the angle has changed. When the above-described array-form light-detecting means and differentiating means are applied to a measurement device using attenuated total reflection, the amount by which a derivative value has changed reflects the amount by which the attenuated total reflection angle (θSP) has changed. Accordingly, based on the amount by which the derivative value has changed, a binding state between a sensing substance and a test substance can be measured (Japanese Patent Application No. 2000-398309 filed by the present applicant). In a measuring method and a measurement device using such attenuated total reflection, a sample solution consisting of a solvent and a test substance is added dropwise to a cup- or petri dish-shaped measurement chip wherein a sensing substance is immobilized on a thin film layer previously formed at the bottom, and then, the above-described amount by which an attenuated total reflection angle (θSP) has changed is measured.

Moreover, Japanese Patent Laid-Open No. 2001-330560 describes a measurement device using attenuated total reflection, which involves successively measuring multiple measurement chips mounted on a turntable or the like, so as to measure many samples in a short time.

When the biosensor of the present invention is used in surface plasmon resonance analysis, it can be applied as a part of various surface plasmon measurement devices described above.

Further, the substrate of the present invention can be used as a biosensor, which has a waveguide structure on the surface of a substrate, for example, and which detects refractive index changes using such a waveguide. The measurement technology of detecting refractive index changes using a waveguide is a technology of detecting an effective refractive index change of a medium adjacent to the waveguide by optical change. The structure of a biosensor of this system is described in column 6, line 31 to column 7, line 47, and Figures 9A and 9B of US Patent No. 6,829,073.

The present invention will be further specifically described in the following examples. However, the examples are not intended to limit the scope of the present invention.

### EXAMPLE

### Example 1: Substrate of the present invention

(1) A pellet of ZEONEX (manufactured by ZEON Corporation) was fused at 240°C, and the fused product was then molded into a prism substrate having a size of 8 mm long x 120 mm wide x 1.5 mm, using an injection molding machine. The prism substrate was attached to a substrate holder of a parallel plate type 6-inch sputtering device (SH-550; manufactured by ULVAC, Inc.), followed by vacuuming (base pressure: 1 x 10⁻³ Pa or less). Thereafter, Ar gas was introduced therein (1 Pa). While rotating the substrate holder (20 rpm), RF power (0.5 kW) was applied to the substrate holder for approximately 9 minutes, so that the prism surface was treated with plasma. Subsequently, introduction of the Ar gas was terminated, followed by vacuuming. The Ar gas was then introduced again (0.5 Pa), and while rotating the substrate holder (10 to 40 rpm), DC power (0.2 kW) was applied to a Cr target with a size of 8 inch for approximately 30 seconds, so as to form a thin Cr film having a thickness of 2 nm. Subsequently, introduction of the Ar gas was terminated, followed by vacuuming. The Ar gas was then introduced again (0.5 Pa), and while rotating the substrate holder (20 rpm), DC power (1 kW) was applied to an Au target with a size of 8 inch for approximately 50 seconds, so as to form a thin Au film having a thickness of 50 nm.
   The thus obtained substrate, on which the thin Au film had been formed, was immersed in a 1 mM aqueous solution of 6-amino-1-octanethiol, hydrochloride (manufactured by Dojindo Laboratories) at 40°C for 1 hour. Thereafter, the resultant substrate was washed with extra pure water 5 times.
(2) Active esterification of CMD (carboxymethyl dextran)
   10 g of 1%-by-weight CMD (manufactured by Meito Sangyo Co., Ltd.; mean molecular weight: 1,000,000; substitution degree: 0.59) solution was dissolved (carboxyl group amount: 5 x 10⁻⁴ mol), and 10 ml of an aqueous solution that contained 1-ethyl-2,3-dimethylaminopropylcarbodiimide (2 x 10⁻⁵ M) was then added to the aforementioned solution, followed by stirring at room temperature for 1 hour.
(3) Binding reaction of CMD to substrate
   1 ml of the active esterified CMD solution prepared in (2) above was added dropwise to the substrate prepared in (1) above, and it was then immobilized at a position corresponding to a radius of 135 mm from the rotation center on the inner cup of a spin-coater (Model 408 (patented); manufactured by Nanotec Corporation) having a hermetically sealed inner cup, such that the tangential direction of an arc became the longitudinal direction of the substrate. It was then spin-coated at 1,000 rpm for 45 seconds, so as to form an actively esterified carboxymethyl dextran thin film on the substrate having an amino group. The reaction was carried out at room temperature for 15 minutes, and the resultant was immersed in a 1 N NaOH aqueous solution for 30 minutes. Thereafter, it was washed with extra pure water 5 times, so as to produce a CMD surface substrate.
(4) Production of avidin-modified substrate
   1 ml of a mixed solution formed by mixing 1-ethyl-2,3-dimethylaminopropylcarbodiimide (400 mM) with N-hydroxysuccinimide (100 mM) at a mixing ratio of 1 : 1 was added dropwise to the surface of the CMD surface substrate produced in (3) above, so that the CMD surface could be coated with the aforementioned mixed solution. The thus coated CMD surface was left at rest at room temperature for 7 minutes, so that the CMD substrate surface could be actively esterified. Subsequently, the substrate surface was washed with a borate buffer, and was eliminated by nitrogen gas.
   100 µl of a solution of 5 mg/ml streptavidin (manufactured by Wako Pure Chemical Industries, Ltd.) was added dropwise to the actively esterified CMD surface substrate, so as to coat the substrate surface with the aforementioned solution. Thereafter, the substrate was immobilized at a position corresponding to a radius of 135 mm from the rotation center on the rotary table of a spin-coater (Model 408 (patented); manufactured by Nanotec Corporation) having a rotary table with a radius of 20 cm, such that the tangential direction of an arc became the longitudinal direction of the substrate. It was then dried by rotating at 500 rpm for 45 seconds in an atmosphere of 23°C and 10% RH. The same operation was repeated 3 times, and the substrate was further left at rest for 30 minutes.
   The thus obtained avidin-modified substrate surface was immersed in a blocking solution at room temperature for 7 minutes. Subsequently, it was immersed in a borate buffer 3 times, and was then immersed in a 1 N sodium hydroxide aqueous solution for 10 minutes. Continuously, it was immersed in a phosphate buffer (pH 7.4) 3 times, so as to produce an avidin-modified substrate that had been subjected to a blocking treatment.
(5) Addition of stabilizer
   1 ml of a 5% dextran (DEX-10 manufactured by Meito Sangyo Co., Ltd.; Mw: 10,000) aqueous solution was added dropwise onto the avidin-modified substrate produced in (4) above, so as to coat the substrate surface with the aforementioned aqueous solution. The substrate was immobilized at a position corresponding to a radius of 135 mm from the rotation center on the rotary table of the spin-coater used in (4) above, such that the tangential direction of an arc became the longitudinal direction of the substrate. It was then rotated at 1,000 rpm for 45 seconds in an atmosphere of 23°C and 10% RH, so as to produce a stabilizer-added substrate.
(6) Evaluation of preservation stability of avidin-modified substrate
   In the case of an avidin-modified substrate, utilizing an avidin-biotin interaction, a biotinylated target protein can be immobilized on the substrate surface while suppressing denaturation. Thus, such an avidin-modified substrate is used as a method of immobilizing a target protein. The sensor surface of the stabilizer-added substrate produced in (5) above is covered with a member made from polypropylene to produce a cell having a size of 1 mm wide (longitudinal direction), 7.5 mm long (horizontal direction), and 1 mm deep. One of the thus produced stabilizer-added substrates was immediately subjected to evaluation of the binding ability of horseradish-derived peroxidase-biotin-XX conjugate (manufactured by Molecular Probes; hereinafter abbreviated as biotinylated HRP). Another stabilizer-added substrate was preserved by enclosing it in nitrogen at 45°C for 7 days, and the binding ability of the biotinylated HRP was then evaluated.
   In order to evaluate such binding ability, the substrate was placed in a surface plasmon resonance device, and an acetate buffer was then added to the cell, followed by leaving at rest for 20 minutes. Thereafter, the cell was filled with a biotinylated HRP solution (100 µg/ml, acetate buffer) for 30 minutes, and it was then substituted with an acetate buffer. A difference between the amount of a resonance signal (RU value) during filling with the acetate buffer before filling the cell with the biotinylated HRP solution and the same above amount after filling the cell with the biotinylated HRP solution was defined as a biotin HRP-immobilized amount. Preservation stability (immobilizing ability-remaining rate) was evaluated after preservation in nitrogen at 45°C for 7 days. As the value is closer to 1.0, it is excellent in terms of preservation stability. The results are shown in Table 1.

### Example 2:

A stabilizer-added substrate was produced by the same method as that applied in Example 1 with the exception that Dex-70 (manufactured by Meito Sangyo Co., Ltd.; Mw: 70,000) was used instead of Dex-10. Thereafter, preservation stability was evaluated.

### Example 3:

A stabilizer-added substrate was produced by the same method as that applied in Example 1 with the exception that Tween 20 (manufactured by Sigma; Mw: approximately 1,200) was used instead of Dex-10. Thereafter, preservation stability was evaluated.

### Example 4:

A stabilizer-added substrate was produced by the same method as that applied in Example 1 with the exception that dextran (manufactured by Sigma; Mw: approximately 2,000,000) was used instead of Dex-10. Thereafter, preservation stability was evaluated.

### Comparative example 1:

A stabilizer-added substrate was produced by the same method as that applied in Example 1 with the exception that dextran (manufactured by Sigma; Mw: 5,000,000 to 40,000,000) was used instead of Dex-10. Thereafter, preservation stability was evaluated.

### Comparative example 2:

A stabilizer-added substrate was produced by the same method as that applied in Example 1 with the exception that CMD (manufactured by Meito Sangyo Co., Ltd.; Mw: approximately 1,000,000) was used instead of Dex-10. Thereafter, preservation stability was evaluated.

### Comparative example 3:

A stabilizer-added substrate was produced by the same method as that applied in Example 1 with the exception that sucrose (manufactured by Wako Pure Chemical Industries, Ltd.; Mw: 342) was used instead of Dex-10. Thereafter, preservation stability was evaluated.

### Comparative example 4:

A stabilizer-added substrate was produced by the same method as that applied in Example 1 with the exception that no stabilizers were added. Thereafter, preservation stability was evaluated.

**Table 1:**

| Stabilizer | Biotinylated HRP-immobilizing ability | | Remarks |
|---|---|---|---|
| | Immediately after formation of film | Immobilizing ability-remaining rate (After preservation at 45°C for 7 days / immediately after formation of film) | |
| Dex-10 | 7,000 RU | 1.0 | The present invention |
| Dex-70 | 6,900 RU | 1.0 | The present invention |
| Tween 20 | 6,600 RU | 0.8 | The present invention |
| Dextran (Mw: 2,000,000) | 3,100 RU | 0.7 | The present invention |
| Dextran (Mw: 5,000,000 to 40,000, 000) | 100 RU | - | Comparative example |
| CMD | 4,000 RU | 0.4 | Comparative example |
| Sucrose | 7,000 RU | 1.0 to 0.4 | Comparative example |
| Without addition of stabilizer | 6,900 RU | 0.4 | Comparative example |

From the results as shown in Table 1, it was demonstrated that the substrate of the present invention to which Compound S had been added, exhibited the same level of performance as that obtained immediately after formation of the film, even after it had been preserved at 45°C for 7 days. On the other hand, when a high-molecular-weight dextran (Mw: 5,000,000 to 40,000,000) was added to such a substrate, the ability of the substrate to immobilize a biotinylated protein almost disappeared. When a low-molecular-weight sucrose was added to such a substrate, a crystal of sucrose was precipitated on the surface thereof after preservation at 45°C for 7 days, and the ability of the substrate to immobilize a biotinylated protein was locally decreased. A substrate, to which CMD that had not been nonionic had been added, did not have a stabilizing effect.

## Claims

1. A substrate which has, on the surface thereof, a physiologically active substance that has been immobilized thereon via a hydrophilic polymer layer formed with hydrophilic polymers, and Compound (Compound S) having a mean molecular weight between 350 and 5,000,000 and having a residue capable of forming a hydrogen bond.

2. The substrate of claim 1 wherein the mean molecular weight of Compound S is between 1,200 and 70,000.

3. The substrate of claim 1 wherein the Compound S is a polysaccharide.

4. The substrate of claim 3 wherein the polysaccharide is composed of 20 to 600 monosaccharides.

5. The substrate of claim 1 wherein the Compound S has a dextran skeleton and has a mean molecular weight between 10,000 and 2,000, 000.

6. The substrate of claim 1 wherein the Compound S is a nonionic compound.

7. The substrate of claim 1 wherein the Compound S has a polyethylene oxide skeleton.

8. The substrate of claim 1 wherein the physiologically active substance is a protein.

9. The substrate of claim 8 wherein the protein is protein A, protein G, avidins, calmodulin, or an antibody.

10. The substrate of claim 1 which has a layer that contains Compound S on a layer that contains the physiologically active substance.

11. The substrate of claim 1 wherein the skeleton of the hydrophilic polymers that form the hydrophilic polymer layer is substantially identical to the skeleton of Compound S.

12. The substrate of claim 1 wherein the ratio of the mean molecular weight of Compound S to the mean molecular weight of the hydrophilic polymers that form the hydrophilic polymer layer is between 0.005 and 0.2.

13. The substrate of claim 1 wherein the physiologically active substance is immobilized on the hydrophilic polymer layer via a covalent bond.

14. The substrate of claim 1 wherein the physiologically active substance is immobilized by activating the hydrophilic polymers that form the hydrophilic polymer layer.

15. The substrate of claim 1 wherein the substrate has a metal film, and the hydrophilic polymer layer binds to the metal film via a self-assembled membrane-forming molecule represented by the following formula A-1:
**HS(CH₂)ₙX** **A-1**
wherein X represents -NH₂, -COOH, -(OCH₂CH₂)ₙOCH₂COOH, -(OCH₂CH₂)ₘO(CH₂)ₙNH₂, -OH, or -(OCH₂CH₂)ₘOH, each n and m each independently represent a number of 1 to 20.

16. The substrate of claim 15 wherein the refractive index of a material for the substrate is between 1.4 and 1.7.

17. The substrate of claim 1 which is used in non-electrochemical detection.

18. The substrate of claim 1 which is used in surface plasmon resonance analysis.

19. A sensor unit comprising the substrate of claim 1.

20. A sensor device comprising the sensor unit of claim 19.

21. A method for producing a substrate, which comprises: a step of allowing a solution containing a physiologically active substance to come into contact with a hydrophilic polymer layer on the surface of a substrate and then drying it, so as to immobilize the physiologically active substance; and a step of allowing an aqueous solution containing Compound S to come into contact with the substrate surface after immobilization of the physiologically active substance.

22. The method of claim 21 wherein the concentration of the solution containing the physiologically active substance is between 0.1 mg/ml and 10 mg/ml.

23. The method of claim 21 wherein the concentration of Compound S in the solution containing said compound is between 0.1% by weight and 5% by weight.

24. An agent for suppressing deactivation of a physiologically active substance, which comprises a compound having a dextran skeleton or a polyethylene oxide skeleton and having a mean molecular weight between 1,200 and 70,000.
